Europäisches Patentamt

European Patent Office

Office européen des brevets

(19)

(11) Publication number: **0 140 476**
**B1**

# EUROPEAN PATENT SPECIFICATION

(12)

(45) Date of publication of patent specification: **28.09.88**

(51) Int. Cl.⁴: **A 61 F 5/44**, A 61 F 5/48

(21) Application number: **84304832.3**

(22) Date of filing: **16.07.84**

(54) Improved incontinence pad.

(30) Priority: **18.07.83 US 515023**

(43) Date of publication of application:
**08.05.85 Bulletin 85/19**

(45) Publication of the grant of the patent:
**28.09.88 Bulletin 88/39**

(84) Designated Contracting States:
**AT BE CH DE FR GB IT LI LU NL SE**

(56) References cited:
**EP-A-0 062 495**
**AT-B- 361 613**
**DE-A-2 656 714**

(73) Proprietor: **E.R. Squibb & Sons, Inc.**
**Lawrenceville-Princeton Road**
**Princeton, N.J. 08540 (US)**

(72) Inventor: **Gegelys, Anthony A.**
**133 Drake Road**
**Somerset New Jersey (US)**

(74) Representative: **Thomas, Roger Tamlyn et al**
**D. Young & Co. 10 Staple Inn**
**London WC1V 7RD (GB)**

EP 0 140 476 B1

Courier Press, Leamington Spa, England.

## Description

The present invention relates to moisture absorbent pads and, more particularly, to an improved moisture absorbent pad primarily designed for use by incontinence patients.

Incontinence is a malady from which a great many elderly and ill individuals suffer. The inability to restrain or control the discharge of waste material from the body, particularly urine, is a problem which often cannot be remedied and, therefore, it is necessary to provide the incontinent individual with a means for containing the discharge, thereby enabling the individual to lead a relatively normal life.

One successful approach to this problem has been the use of incontinence garments such as briefs or the like, which can be washed and reused. Such garments are provided with a pocket-like structure into which a disposable moisture absorbent incontinence pad can be inserted. The pad, once it becomes moisture laden, is removed from the garment and a new pad is substituted in its place.

Incontinence pads normally include a layer of moisture absorbent core material such as wood pulp, tissue wadding, foams, non-wovens, batting, or the like. The moisture absorbent core material is surrounded by moisture permeable cover sheet which contains the core material and maintains the integrity of the pad. The ends of the cover sheet are usually sealed, by crimping or the like, such that the moisture absorbent core material is completely enclosed by the cover sheet.

In order to increase the absorbency of the core material, the core material may be divided into layers and a moisture diffusable layer interposed therebetween. The moisture diffuser layer may include alternating compressed and stretched zones of tissue or the like and acts to spread the moisture more evenly through the pad to increase its capacity.

Incontinence pads of the type described above suffer from several disadvantages which the improved incontinence pad of the present invention is designed to overcome. One of the problems relates to the tendency of moisture absorbed by the core material to leak from the sides of the pad. This problem becomes particularly acute when the moisture absorbent core material is saturated.

Another problem relates to the tendency of moisture absorbed by the core material to migrate back through the upper surface of the cover sheet towards the wearer, resulting in the pad having a wet, uncomfortable feel.

In this connection, European Patent Application EP-A-62495 describes an incontinence pad in which absorbent sheet material is removably enclosed in a sheath. The sheath may be of nonwoven fabric made from polyester, and in an embodiment not described in any detail, the part forming the back and sides of the sheath may be replaced by a water-impervious sheet. The absorbent sheet material may be a multilayer material, at least one layer of which is provided with openings Composites including layers of tissue are described and modified starches can be used as the absorbent material.

According to the present invention, there is provided an incontinence pad comprising a cover sheet having a first section of a nonwoven moisture permeable polyester fiber of from 0.25 to 2 ounces per square yard (8.5 to 68 g/m²), and a second section of moisture impermeable polyethylene film of from 0.5 mils (12.7 μm) to 3 mils (76.2 μm) thickness, said sections of said cover sheet being joined to form an enclosure, a core layer of moisture absorbent fluffed wood pulp material having a thickness of from 0.15 inches (3.8 mm) to 1.5 inches (38 mm), a uni-directional moisture barrier layer of substantially uniform thickness comprising a laminate containing a polymer that expands when wetted, said barrier layer having a plurality of apertures therethrough, said apertures having a given diameter prior to the expansion of said polymer to facilitate the passage of liquid through said barrier layer to said absorbent layer and constituting from 15% to 35% of the surface area of said barrier layer prior to contact with moisture, the diameter of said apertures decreasing as said polymer expands so as to restrict the passage of liquid back through said barrier layer towards said first section, and a tissue material wicking layer of 1 mil (25.4 μm) to 3 mils (76.2 μm) thickness interposed between said cover sheet and said barrier layer.

Preferred forms of the invention will now be described.

An incontinence pad comprises a cover sheet having a moisture permeable top section and a moisture impermeable side and bottom section. Means are provided for affixing the edges of the interior surface of the moisture impermeable section to the edges of the exterior surface of the moisture permeable section to form an enclosure. A layer of moisture absorbent core material is situated within the enclosure. A uni-directional moisture barrier layer is interposed between the permeable cover section and the absorbent layer. This uni-directional moisture barrier layer includes a plurality of apertures therethrough.

The barrier layer is a laminate of a "super absorbent" polymer and a base or carrier of paper or the like. Suitable commercially available "super absorbent" polymers include starches, acrylics, modified celluloses, gums and the like. The polymer material may be coated onto a paper base or sandwiched between layers of paper to form the laminate. The "super absorbent" polymer, once it becomes wet, will gel and expand. The apertures in the barrier layer facilitate the movement of moisture from the moisture-permeable section of the cover sheet, through the barrier layer, and into the moisture absorbent core material. As the polymer becomes wet and gels, it expands, thereby reducing the size of the apertures, and preventing the moisture from moving back through the barrier layer from

the moisture absorbent core material to the cover sheet. Thus, the apertured layer acts to retain moisture in the absorbent core material by functioning as a uni-directional moisture barrier.

The barrier layer is of a substantially uniform thickness resulting in a pad that absorbs moisture in a predictable manner from one pad to the next unlike prior products where the "super absorbent" is randomly dispersed in the core layer or contained in pockets. Also, the apertures which decrease in size as more moisture contacts the barrier layer provides a way-of controlling the amount of moisture reaching the absorbent core and prevents this moisture, when the core becomes saturated, from migrating back through the barrier layer to the liquid permeable cover and the wearer.

The moisture permeable and moisture impermeable sections of the cover sheet form an enclosure. Preferably, at least one end of the enclosure is releasable sealed to permit the material therein, which is bio-degradable, to be removed from the cover sheet and disposed of.

The moisture permeable section of the cover sheet is composed of a non-woven polyester material. The moisture impermeable section is composed of polyethlene film.

A wicking layer is interposed between the cover section and the barrier layer. This wicking layer functions to more uniformly disperse the fluid passing through the permeable cover sheet onto the barrier layer. The wicking layer comprises a layer of moisture permeable tissue material.

Fig. 1 is an exploded isometric view of a preferred incontinence pad of the present invention;

Fig. 2 is a perspective view of the pad of Fig. 1;

Fig. 3 is a plan view of the the pad of Fig. 1, showing the layers thereof broken away;

Fig. 4 is a longitudinal sectional view of the pad taken along the line 4-4 of Fig. 2; and

Fig. 5 is a transverse sectional view of the pad taken along the line 5-5 of Fig. 2.

As shown in the figures, an incontinence pad of the present invention includes a cover sheet 10 formed of a rectangular liquid permeable top section 10a, and a liquid impermeable side and bottom section 10b. Liquid permeable section 10a is composed of non-woven material made of polyester fibres, which permits the passage of fluid therethrough. The liquid impervious section 10b is formed of polyethylene.

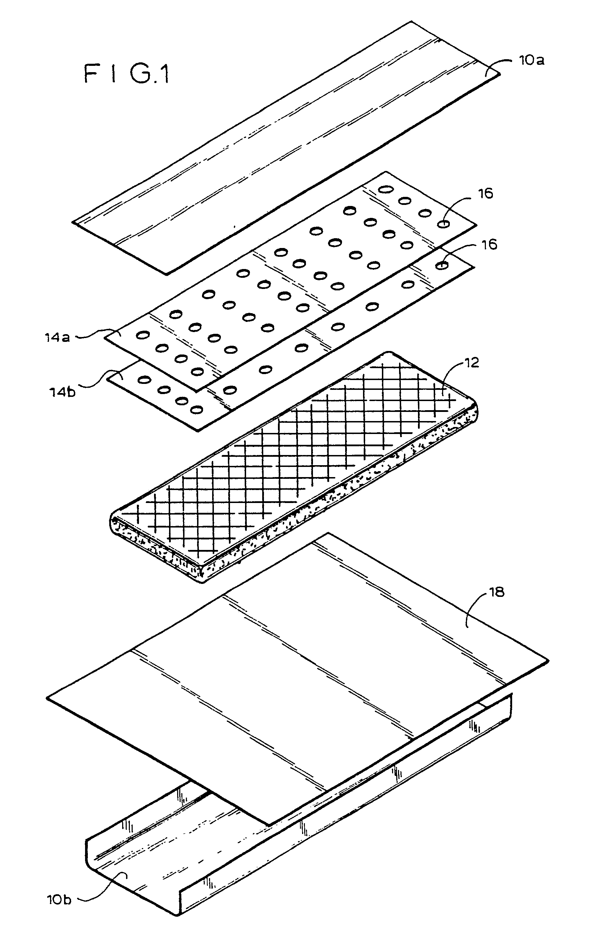

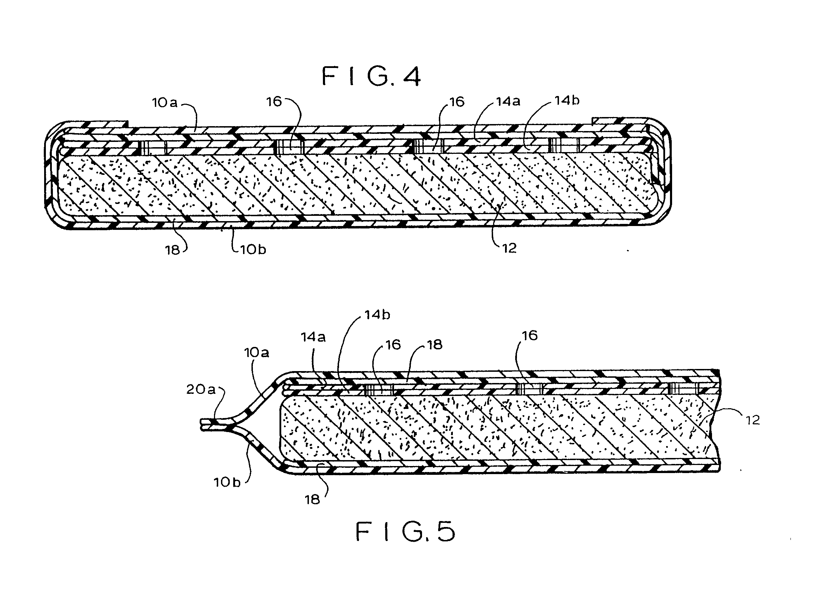

As illustrated in Figs. 1 and 4, during manufacture of the pad, the end portions of the moisture impermeable section 10b are folded upwardly, to form the sides of the pad, and then inwardly so as to overlap the edges of the liquid permeable section 10a. The edges of the interior surface of the liquid impermeable section 10b overlap the exterior edges of the liquid permeable section 10a by 0.25 inches (6.4 mm) to 0.50 inches (12.7 mm). The overlapping surfaces of the liquid impermeable section 10b are then affixed to the exterior edges of the liquid permeable section 10a by means of adhesive or the like.

In this manner, the two sections which make up the cover sheet form an enclosure which surrounds the remaining elements of the pad, so as to maintain the integrity thereof. The use of liquid impervious material for the bottom and sides of the pad and in an overlapping relationship with the edges of the liquid permeable top section of the pad, prevents moisture from leaking out the sides or bottom of the pad when the pad is saturated.

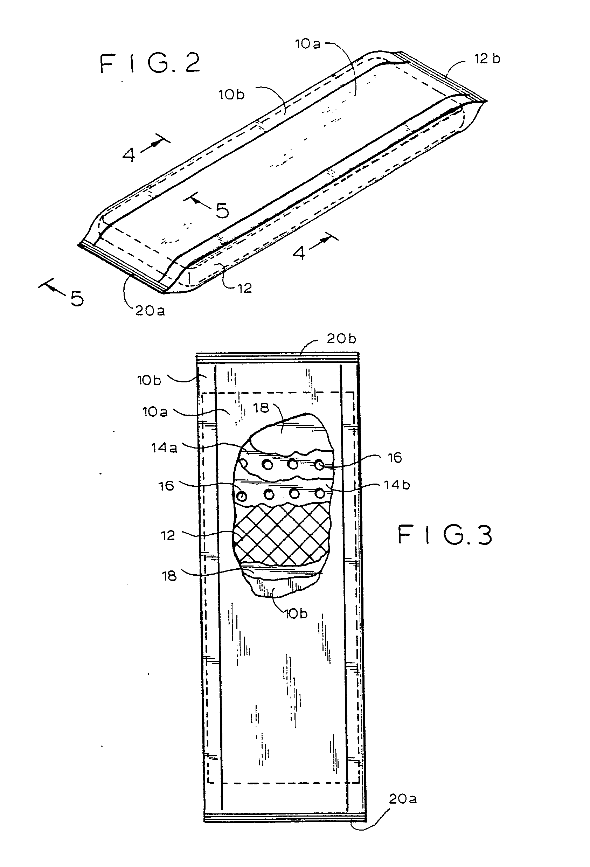

In the interior of the enclosure formed by cover sheet 10 is a relatively thick layer of moisture absorbent fluffed wood pulp core material 12. Preferably, the exterior of layer 12 is embossed. This embossing enhances the ability of the core material to wick and disperse moisture and also enhances the integrity of the core layer, particularly when wet. As shown in Figs. 1 and 3, the embossing may be in a diamond-like pattern.

Situated adjacent the top surface of the moisture absorbent layer 12 is a planar, rectangularly shaped uni-directional moisture barrier 14 of uniform thickness. Barrier layer 14 is situated in alignment with the moisture permeable section 10a of cover sheet 10 and functions to retain moisture in the absorbent core layer 12. The "super absorbent" polymer constituent of barrier layer 14 gels and expands when wet. Thus, this layer will act to block the transfer of moisture therethrough. Barrier 14, if formed of an uninterrupted laminate, would gradually restrict the amount of moisture passing therethrough and, finally, after being completely gelled and expanded, would effectively block any moisture from passing into moisture absorbent material 12.

To enhance the amount of fluid, i.e. urine, which can pass through uni-directional barrier layer 14, barrier layer 14 is provided with a large number of apertures 16. The shape, size, and number of apertures can be varied according to the gelling and expansion characteristics of the particular "super absorbent" polymer employed. As layer 14 gradually undergoes the transition which results in gelling and expanding, the swelling of the "super absorbent" polymer substantially decreases the size of the apertures. Thus, the passage of moisture through the barrier layer is gradually reduced, but much more gradually than if no apertures were present.

Accordingly, barrier 14 will act to retain moisture within the absorbent core material by permitting a substantial amount of moisture to pass through it to absorbent core layer 12, but, thereafter, preventing any substantial amount of liquid from passing from absorbent core layer 12 back through barrier 14 to the liquid permeable section 10a of the cover sheet and, thus, back to the wearer. The result is that the top of the pad which is composed of the liquid permeable section 10a, will be more comfortable to the wearer because it will not have a wet feel.

Uni-directional barrier layer 14 may be a single sheet of material, several sheets arranged so that the apertures 16 are aligned as note sheets 14a and 14b in the figures, or a single sheet bent or folded in such a manner that apertures 16 are aligned. preferred materials for barrier layer 14 are a laminate containing a "super absorbent" starch polymer known as DWAL (trademark of Dow Chemical) and a laminate containing a "super absorbent" modified acrylic polymer known as Gelok 4000 (trademark of Gelok International).

Moisture absorbent core layer 12 and uni-directional barrier layer 14 are surrounded by a tissue material wicking layer 18. This wicking layer functions to more uniformly disperse the fluid, i.e. urine, passing through permeable cover sheet 10a onto uni-directional barrier layer 14. Thus, wicking layer 18 ensures that the "super absorbent" polymer component of barrier layer 14 will gel and expand more uniformly across the entire surface of layer 14.

After barrier layer 14 is situated on moisture absorbent core layer 12 and wicking layer 18 is wrapped therearound, the interior of the pad is placed on the moisture impermeable section 10b of the cover sheet. The moisture permeable section 10a is then situated on top of layer 18 in alignment with barrier layer 14 and the edges of the liquid impermeable section 10b are affixed to the exterior surface of the edges of liquid permeable section 10a, by adhesive or the like, as described above.

To finish the pad, the ends 20a, 20b thereof are sealed such as by crimping, heat sealing, ultrasonic welding, gluing, or the like, as illustrated in Fig. 2. Preferably, at least one of ends 20a and/or 20b is sealed in a releasable manner such that the pad interior, which is composed of layers 12, 14, and 18, can be removed from cover sheet 10 for disposal purposes. In this regard, it should be noted that layers 12, 14, and 18 are composed of bio-degradable materials which can be flushed down a toilet or the like. However, sections 10a and 10b of the cover sheet are not composed of bio-degradable material and, thus, the interior of the pad must be removed from the cover sheet prior to flushing, if disposal in this manner is desired.

It should now be appreciated that the incontinence pad eliminates the problems of, liquid leakage from the sides of the pad, thereby eliminating the possibility of embarrassing wet spots on the wearer's clothing. Moreover, the uni-directional moisture barrier prevents passage of liquid back to the top of the pad and, thus, the uncomfortable wet feel associated with prior art incontinence pads.

Further details of the incontinence pad are as follows. Liquid permeable cover sheet 10a is a non-woven polyester material of from 0.25 to 2 ounces per square yard (8.5 to 68 g/m²), most preferably 0.5 ounces per square yard (17 g/m²). Liquid impermeable film 10b is a polyethylene film of from 0.5 mils (12.7 µm) to 3 mils (76.2 µm), most preferably at about 1 mil (25 µm) thickness. Wicking layer 18 is tissue material at from 1 to 3 mils (25.4 to 76.2 µm), most preferably about 2 mils (50.8 µm) thick. Uni-directional barrier layer or layers 14 are preferably of sufficient thickness and include an amount of "super absorbent" polymer so as to absorb from 30 to 100 times their weight in urine. Apertures 16 are dimensioned so as to occupy from 15% to 35% of the surface area, most preferably about 25% of the surface area, of barrier layer or layers 14 prior to contact with moisture. Moisture absorbent core material 12 is a fluffed wood pulp of 0.15 inches (3.8 mm) to 1.5 inches (38 mm) thick, most preferably 0.25 inches (6.4 mm) to 0.5 inches (12.7 mm).

The exterior surface of liquid impermeable layer 10b may include one or more adhesive strips to aid in securing the pad to a garment.

**Claims**

1. An incontinence pad comprising a cover sheet (10) having a first section (10a) of a non-woven moisture permeable polyester fiber of from 0.25 to 2 ounces per square yard (8.5 to 68 g/m²), and a second section (10b) of moisture impermeable polyethylene film of from 0.5 mils (12.7 µm) to 3 mils (76.2 µm) thickness, said sections of said cover sheet being joined to form an enclosure, a core layer (12) of moisture absorbent fluffed wood pulp material having a thickness of from 0.15 inches (3.8 mm) to 1.5 inches (38 mm), a uni-directional moisture barrier layer (14) of substantially uniform thickness comprising a laminate containing a polymer that expands when wetted, said barrier layer having a plurality of apertures (16) therethrough, said apertures (16) having a given diameter prior to the expansion of said polymer to facilitate the passage of liquid through said barrier layer (14) to said absorbent layer (12) and constituting from 15% to 35% of the surface area of said barrier layer prior to contact with moisture, the diameter of said apertures (16) decreasing as said polymer expands so as to restrict the passage of liquid back through said barrier layer (14) towards said first section (10a), and a tissue material wicking layer (18) of 1 mil (25.4 µm) to 3 mils (76.2 µm) thickness interposed between said cover sheet (10) and said barrier layer (14).

2. A pad according to claim 1, wherein at least one end of said enclosure is releasably secured. ·

3. A pad according to claim 1, wherein said barrier layer (14) is a plurality of apertured sheets, said apertures (16) being substantially aligned, each sheet being of substantially uniform thickness and comprising a laminate of a base or carrier and a polymer that expands when wetted.

4. A pad according to claim 3, wherein said polymer is a starch polymer.

5. A pad according to claim 3, wherein said polymer is an acrylic polymer.

6. A pad according to claim 1, wherein said barrier layer (14) is an apertured sheet of substantially uniform thickness folded one or more times such that the apertures (16) are substantially aligned, said sheet comprising a laminate of a base or carrier and a polymer that expands when wetted.

7. A pad according to claim 6, wherein said polymer is a starch polymer.

8. A pad according to claim 6, wherein said polymer is an acrylic polymer.

**Patentansprüche**

1. Inkontinenz-Unterlage mit einer Abdecklage (10), die ein erstes Teil (10a) aus einem feuchtigkeitsdurchlässigen Polyesterfaservliesstoff mit 0,25 bis 2 Ounces pro Square Yard (8,5 bis 68 g/m²) und ein zweites Teil (10b) aus feuchtigkeitsundurchlässiger Polyethylenfolie mit einer Dicke von 0,5 mil (12,7 µm) bis 3 mil (76,2 µm) hat, wobei die Teile der Abdecklage zur Bildung einer Hülle verbunden sind, welche eine Kernschicht (12) aus feuchtigkeitsabsorbierendem, flockigem Zellstoffmaterial aufweist, die eine Dicke von 0,15 Inch (3,8 mm) bis 1,5 Inch (38 mm) hat, eine in einer Richtung wirkende Feuchtigkeitssperrschicht (14) mit im wesentlichen gleichförmiger Dicke aufweist, die ein Laminat aufweist, das ein Polymer enthält, das sich beim Befeuchten expandiert, wobei die Sperrschicht eine Mehrzahl von durchgehenden Öffnungen (16) hat, wobei die Öffnungen (16) einen bestimmten Durchmesser vor der Expansion des polymers haben, um den Flüssigkeitsdurchgang · durch die Sperrschicht (14) zu der absorbierenden Schicht (12) zu unterstützen, und dievon 15% bis 35% des Flächenbereichs der Sperrschicht vor dem Kontakt mit Feuchtigkeit bilden, wobei der Durchmesser der Öffnungen (16) kleiner wird, wenn das Polymer expandiert, so daß der Rückfluß der Flüssigkeit durch die Sperrschicht (14) zu dem ersten Teil (10a) begrenzt wird, und welche eine Gewebematerialdochtschicht (18) mit einer Dicke von 1 mil (25,4 µm) bis 3 mil (76,2 µm) aufweist, die zwischen der Abdecklage (10) und der Sperrschicht (14) angeordnet ist.

2. Unterlage nach Anspruch 1, bei der wenigstens ein Ende der Hülle lösbar befestigt ist.

3. Unterlage nach Anspruch 1, bei der die Sperrschicht (14) von einer Mehrzahl von mit Öffnungen versehenen Lagen gebildet wird, wobei die Öffnungen (16) im wesentlichen zueinander fluchten, und wobei jede Lage eine im wesentlichen gleichförmige Dicke hat und ein Laminat aus einer Unterlage oder einem Träger und einem Polymeren aufweist, das beim Befeuchten expandiert.

4. Unterlage nach Anspruch 3, bei der das Polymer ein Stärkepolymer ist.

5. Unterlage nach Anspruch 3, bei der das polymer ein Acrylpolymer ist.

6. Unterlage nach Anspruch 1, bei der die Sperrschicht (14) eine mit Öffnungen versehene Lage mit im wesentlichen gleichförmiger Dicke ist, die ein oder mehrmals gefaltet ist, so daß die Öffnungen (16) im wesentlichen fluchten, wobei die Lage ein Laminat aus einer Unterlage oder einem Träger und einem Polymeren aufweist, das beim Befeuchten expandiert.

7. Unterlage nach Anspruch 6, bei der das Polymer ein Stärkepolymer ist.

8. Unterlage nach Anspruch 6, bei der das Polymer ein Acrylpolymer ist.

**Revendications**

1. Rembourrage pour incontinents, comprenant une feuille de couverture (10) ayant une première section (10a) de fibre de polyester non tissée, perméable à l'humidité, de 8,5 à 68 grammes par mètre carré, et une seconde section (10b) de film de polyéthylène imperméable à l'humidité, de 12,7 µm à 76,2 µm d'épaisseur, lesdites sections de ladite feuille de couverture étant réunies pour former une enceinte, une couche centrale (12) de pâte de bois rendue duveteuse, absorbant l'humidité, de 3,8 à 38 millimètres d'épaisseur, une couche (14) faisant écran à l'humidité dans un seul sens, d'épaisseur sensiblement uniforme, comprenant un stratifié contenant un polymère qui gonfle au mouillage, ladite couche-écran étant percée de plusieurs ouvertures (16), lesdites ouvertures (16) étant de diamètre donné avant le gonflement dudit polymère pour faciliter le passage du liquide, à travers ladite couche-écran (14), dans ladite couche absorbante, et constituant de 15 % à 35 % de la superficie de ladite couche-écran avant contact avec l'humidité, le diamètre desdites ouvertures (16) diminuant à mesure que ledit polymère gonfle de manière à limiter le retour du liquide à travers ladite couche-écran (14) vers ladite première section (10a), et une couche-buvard (18) en tissu, de 25,4 µm à 76,2 µm d'épaisseur, interposée entre ladite feuille de couverture (10) et ladite couche-écran (14).

2. Rembourrage selon la revendication 1, dans lequel au moins une extrémité de ladite enceinte est fixée de manière amovible.

3. Rembourrage selon la revendication 1, dans lequel ladite couche-écran (14) comprend plusieurs feuilles à ouvertures, lesdites ouvertures (16) étant sensiblement alignées, chaque feuille étant d'épaisseur sensiblement uniforme et comprenant un stratifié d'une base ou support et d'un polymère qui gonfle au mouillage.

4. Rembourrage selon la revendication 3, dans lequel ledit polymère est un polymère d'amidon.

5. Rembourrage selon la revendication 3, dans lequel ledit polymère est un polymère acrylique.

6. Rembourrage selon la revendication 1, dans lequel ladite couche-écran (14) est une feuille à ouvertures, d'epaisseur sensiblement uniforme, pliée une ou plusieurs fois de telle sorte que les ouvertures (16) sont sensiblement alignées, ladite feuille comprenant un stratifié d'une base ou support et d'un polymère qui gonfle au mouillage.

7. Rembourrage selon la revendication 6, dans lequel ledit polymère est un polymère d'amidon.

8. Rembourrage selon la revendication 6, dans lequel ledit polymère est un polymère acrylique.

F I G.1

10a

16

16

14a

14b

12

18

10b

F I G. 2

F I G.3

# F I G. 4

10a  16  16  14a  14b

18  10b  12

# F I G. 5

14b
14a  16  18  16
10a
20a
12
10b
18